Europäisches Patentamt

European Patent Office    (11) Publication number: **0 316 838**

Office européen des brevets                          **A1**

(12)                    **EUROPEAN PATENT APPLICATION**

(21) Application number: 88118928.6          (51) Int. Cl.⁴: **A61K 9/06**

(22) Date of filing: 14.11.88

Claims for the following Contracting States: ES + GR.

(30) Priority: 18.11.87 IT 2268287

(43) Date of publication of application:
24.05.89 Bulletin 89/21

(84) Designated Contracting States:
AT BE CH DE ES FR GB GR IT LI LU NL SE

(71) Applicant: **FARMA RESA S.r.l.**
**Via Como, 5**
**I-22063 Cantu' Como(IT)**

(72) Inventor: **Conte, Ubaldo**
**Via Treviglio, 6**
**I-21052 Busto Arsizio (Varese)(IT)**
Inventor: **La Manna, Aldo**
**Via Lanfranco, 3**
**I-27100 Pavia(IT)**
Inventor: **Colombo, Paolo**
**Via Magenta, 12**
**I-27100 Pavia(IT)**
Inventor: **Saettone, Marco F.**
**Via Giusti, 24**
**I-55049 Viareggio (Lucca)(IT)**
Inventor: **Gazzaniga, Andrea**
**Via Emilia, 16**
**I-27046 Santa Giulietta (Pavia)(IT)**

(74) Representative: **Gervasi, Gemma**
**NOTARBARTOLO & GERVASI Srl Viale**
**Bianca Maria 33**
**I-20122 Milan(IT)**

(54) Tablet for opthalmic use with controlled release of the active substance.

(57) A tablet for ophthalmic use with controlled release of the active substance applicable both in the human and veterinary field. In said tablet a surface portion (a) is coated with polymer material (b) which is impermeable and/or insoluble or only slowly soluble in water, and can be further coated with bioadhesive polymer material (c).

The tablet, which can be positioned in the conjunctival sac of he eye and remain there for a programmed time period, enables the number of medicament administrations to be reduced and is usable in particular for prolonged therapeutic treatment.

It is furthermore described a preparation method.

FIG. I

## TABLET FOR OPHTHALMIC USE WITH CONTROLLED RELEASE OF THE ACTIVE SUBSTANCE

### Prior art

The difficulties in administering medicaments for ophthalmic use are well known. In this respect, introducing a foreign body in the eye (such as a drop of collyrium) results as reflex action in a considerable increase in lacrimal secretion and an increase in reflex blinking in an attempt to expel the "foreign body".

This process results in rapid elimination of the administered active principle with the result that a therapeutically effective quantity of active principle can be maintained at the site of application only for a very limited time period.

In order to increase the period for which a large quantity of medicament is maintained in the eye, pharmaceutical forms other than water-based collyria have been developed, such as water-based collyria containing viscosity-increasing substances and/or oily or lipophil solutions difficult to remove by the lacrimal aqueous liquid.

These forms have however various drawbacks and in any event require numerous applications during the course of the day. In addition, it is particularly difficult to maintain high medicament levels in situ during the night.

To obviate these difficulties therapeutic systems for ophthalmic use have been developed in recent years which, when inserted into the conjunctival sac of the eye, release the active principle contained in them at a constant rate for a prolonged time period.

The therapeutic systems currently available for ophthalmic use can be divided into two classes:
- matrix systems
- reservoir systems.

Both hydrogel and soluble ocular inserts pertain to the first class.

Hydrogel inserts consist mainly of hydrophil polymer material which, when in contact with the lacrimal liquid, swells slowly and is transformed into hydrogel.

If medicaments are vehicled in the polymer material, high medicament levels can be obtained in the application site with this type of therapeutic system.

However, this system is not always well tolerated, because of the large degree of swelling of the polymer material consequent on its hydration.

Soluble ocular inserts consist essentially of hydrophil polymer materials which when in contact with the lacrimal liquid absorb water to dissolve slowly and increase the viscosity of the lacrimal liquid.

With systems of this type the vehicled active principle (such as pilocarpine, hydrocortisone, gentamicin etc.) is maintained for a longer period in contact with the absorption site, mainly because of the increased viscosity of the lacrimal liquid.

This system has however the drawback of not allowing the rate of release of the active principle to be controlled and of resulting in large variations between individuals.

One reservoir system of considerable interest is the "Ocusert" system, in which the medicament is contained in a deposit surrounded by a polymer membrane which controls its release rate.

Although this is a technologically advanced system, it is of high cost and has other drawbacks such as being disturbing to the patient and tends to be expelled spontaneously during sleep.

A system for the controlled release of active substances has been patented under European patent application No. 86116649.4 in the name of COLOMBO Paolo et al.

This system consists of a core comprising the active substance and a polymer material having a high degree of swelling, plus a support consisting of an insoluble polymer material which partly coats said core.

In this system the primary factor which controls the release of the active substance is the swelling of the polymer material in the core.

### Summary of the invention

According to the present invention, we have now discovered a tablet for ophthalmic use which enables active substances of different solubility to be vehicled and provides a therapeutic response for a prolonged time period, thus obviating the drawbacks of the known art.

Said tablet consists of:

- a matrix (a) of defined geometrical form containing the active substance and suitable excipients and/or additives able to give the mixture the characteristics required for its compression;
- a polymer coating (b) applied to a portion of the surface of said matrix such as to make the coated matrix portion impermeable or slowly permeable;
- a layer (c) of bioadhesive polymer material applied to said coating (b).

The layer (c) of bioadhesive polymer material can also be absent from the embodiment, depending on the characteristics of said coating (b). In this respect, if the polymer material used for the coating (b) possesses sufficient bioadhesive properties the two required functions are both performed by this coating.

## Detailed description of the invention

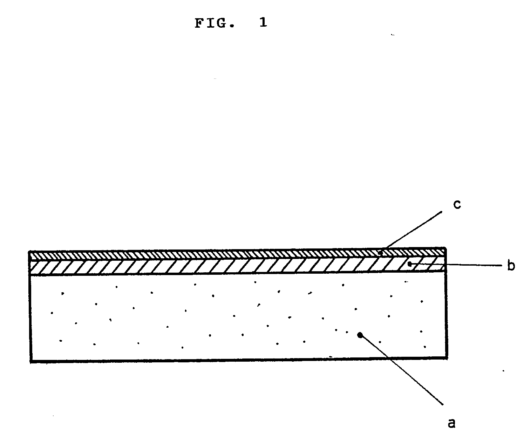

A schematic representation of the tablet embodiment according to the present invention is shown in Figure 1 in which (a) indicates the tablet matrix, (b) indicates the polymer coating applied to one face of the matrix, and (c) indicates the layer of bioadhesive polymer material. This embodiment limits the release of the medicament from the matrix only to that part not covered by the impermeable polymer material, so increasing the period of activity and considerably reducing the quantity of active substance normally absorbed by the palpebral conjunctiva to produce undesirable systemic effects.

The therapy is therefore optimized in that the active substance is released mainly towards the corneal surface, to maintain effective therapeutic concentrations for a prolonged time, to obtain in the meantime a reduction in the systemic absorption which takes place transconjunctivally.

The tablets can also be of very different shape and size from that shown in the figure which is given by way of non-limiting example of the invention.

In this respect, their shape and size depend on the type of subject (human or animal) treated, it being apparent that their optimum geometry must take account of the eye size and its geometrical characteristics (curvature, etc.). In the cylindrical embodiment for human use the tablet has the following dimensions:
- diameter: from 2 to 8 mm
- height: from 0.1 to 2mm.

In addition to the described geometrical form, tablets of lenticular, oblong or other shapes can be produced, to also take account of the pathological symptoms to be treated and to make the therapeutic system less irritating to the eye.

The surface to be covered can also be slightly concave to enable the covering solution or suspension to more easily remain in situ.

In forming the tablet for ophthalmic application, many aspects must be taken into account, and in particular:
- the physico-chemical characteristics of the active substance (solubility, solution pH, distribution coefficient etc.);
- the characteristics of the polymer material (solubility, gelling properties on contact with water, bioadhesiveness).

In particular, in preparing the matrix (a), excipients and/or lubricating, antiadhesion and binding additives are added in addition to the active substance, in order to give the mixture favourable characteristics under compression and/or compaction.

A very large number of active substances can be used, such as pilocarpine, beta-blocking agents, steroid and non-steroid antiinflammatories, antibiotics, antivirals and their derivatives (salts, esters etc.), used singularly or in association.

Biocompatible and biodegradable polymers of different degrees of hydrophilism and solubility in water of aqueous fluids can be used as excipients. Preferably but not limitatively, polyvinyl alcohols and polyacrylic acid of various molecular weights, hydroxymethylcellulose, hydroxypropylcellulose and cellulose derivatives of different molecular weights are used.

Paraffins, magnesium stearate and glycerol mono-bi-tripalmito-stearate are used as additives.

The matrices (a) are prepared using reciprocating or rotary compressing machines by methods well known to the experts of the art, or can be prepared by more complex and precise apparatus using suitable punches in relation to the geometrical form required.

The compression can be conducted by applying a pressure variable between 300 and 5000 kg/cm$^2$ in relation to the required active substance release rate and the sensitivity of the mixture to compressive force.

The matrices obtained in this manner are covered on one face by applying a coating (b) of polymer material which is impermeable or only slowly permeable to aqueous liquids. This coating can be applied by atomization or deposition of a solution or suspension of a suitable polymer material or other substances.

Polymer materials usable for the layer (b) are chosen from polymethylmethacrylates, ethylcellulose, hydroxypropylcellulose and derivatives, and esters such as diethyl phthalate, ethyl acetate and butyl acetate can be used in mixture with the former.

In order to be able to recognise which is the coated face at the time of the therapeutic application, biocompatible colouring and/or opacifying substances can be added in accordance with the known art.

If the applied layer of polymer material does not have sufficient bioadhesiveness towards the palpebral mucous membrane, a second layer (c) of bioadhesive polymer material is applied over the layer (b) to give the system the necessary adhesiveness.

Suitable bioadhesive polymer materials include natural and/or synthetic polycarboxylic acids, alginic acid, carboxypolymethylene, hyaluronic acid, galacturonic acid and other natural or synthetic materials.

As in the case of the layer (b), the layer (c) is also applied by atomization or deposition of a solution or suspension of said polymer materials or other substances.

The solution is prepared preferably using alcohols or ketones as solvents, and the suspension is prepared preferably using water as suspending agent.

The polymer material content is between 1 and 40% by weight in all cases.

Atomization is effected by spraying said solution or suspension using an air jet or an airless gun.

Deposition is effected by gravity fall, metering a defined quantity of solution or suspension.

In addition to application by atomization or deposition of a polymer solution, layers (b) and (c) can be applied by compression.

The tablets according to the invention enable the quantity and rate of release of the active substance to be varied in that lipidic additives can be added to the mixture to reduce the rate at which the medicament diffuses and dissolves. Further control can be obtained by limiting that surface area of the tablets which is not coated with impermeable polymer material.

In addition the presence of the bioadhesive polymer layer enables the tablet to remain in situ, so ensuring correct application within a limited region of the eye and a prolongation of activity.

The following example of the preparation of tablets according to the invention is given by way of non-limiting illustration.

EXAMPLE 1

Preparation of tablets for ophthalmic use containing pilocarpine nitrate as active substance

1st stage: Preparation of the matrix (a).

The following product quantities were used to prepare 1000 matrices:

| | |
|---|---|
| Pilocarpine nitrate | 1300 mg |
| Glycerol mono-di-tripalmito-stearate (Precirol ATO 5 Gattefossè) | 2600 mg |
| Lactose | 2500 mg |
| Hydroxypropylmethylcellulose (HPC M.W 100,000 - Jansen B) | 9600 mg |

The Precirol was melted in a porcelain dish over a water bath at a temperature less than 45° C and the pilocarpine nitrate was added while stirring, to obtain a homogeneous mass which was allowed to cool to ambient temperature and then passed through a 50 mesh stainless steel sieve. Lactose previously sieved through a 50 mesh sieve was added to the granular product obtained and the mixture was stirred until homogeneous. The mixture was then transferred into a container and the previously sieved hydroxypropylcellulose was added, and the mixture again stirred until homogeneous.

The granulate obtained was then compressed using flat punches of 5.5 mm diameter and operating at different pressure levels from 500 to 3000 kg/cm$^2$.

The geometrical characteristics of the matrix obtained are as follows:

4

| diameter | 5.5 mm |
|---|---|
| thickness | 0.6 mm |
| total surface area | 57.86 mm². |
| surface area of one face | 23.76 mm². |

## 2nd stage: coating one face of the matrix (a) with the layer (b)

A series of matrices prepared in accordance with the preceding stage were coated on one face with a solution of the following composition:

| Acrylic copolymer (Eudragit RS - Rohm Pharma) | 20 g |
|---|---|
| Diethylphthalate | 2.0 g |
| Ethyl acetate | 12 g |
| Butyl acetate | 13 g |
| Toluene | 41 g |

One drop of the aforesaid solution was deposited on that face to be coated, in impermeable film then being formed by spontaneous evaporation of the solvent.

The solution was applied a second time to obtain a film of thickness between 10 and 30 microns.

## 3rd stage: coating layer (b) with a layer (c) of bioadhesive polymers

A layer (c) of bioadhesive polymer material was applied to that matrix face previously coated with the impermeable layer (b), using the aforesaid procedure.

A solution was used having the following composition:

| Carboxypolymethylene (carbopol 940, Goodrich - USA) | 5 g |
|---|---|
| 95° ethanol | 95 g |

A layer (c) of negligible thickness was obtained which had good bioadhesive properties when in contact with the palpebral mucous membrane.

## Evaluation of activity of the tablets of Example 1

The miotic activity determined by the release of the active substance from the tablets of Example 1 was evaluated on the rabbit, specifically using for each trial 8 male albino rabbits of weight 3 kg.

The tablets were placed in the lower conjunctival sac of one eye of the rabbit, keeping the other eye as control.

The trial was conducted both using tablets coated with layers (b) and (c) using uncoated tablets.

Tables 1 and 2 show the variation of pupil diameter $\Delta\phi$ (difference with respect to initial value) with time and the area under the activity/time curve (AUC) for the uncoated tablets and for the tablets coated with layers (b) and (c) respectively.

TABLE 1

| Uncoated tablets | |
| --- | --- |
| TIME (minutes) | $\Delta\emptyset$ (mm) |
| 0 | 0 |
| 10 | .5833 |
| 30 | 1.416 |
| 45 | 2 |
| 60 | 1.666 |
| 90 | 1.833 |
| 120 | 1.916 |
| 150 | 2.083 |
| 180 | 2.333 |
| 210 | 2.083 |
| 240 | 1.75 |
| 270 | 1.5 |
| 300 | 1.5 |
| 330 | 1.25 |
| 360 | 1.416 |
| 390 | .75 |
| 420 | .3333 |
| Maximum pupil diameter variation ($\Delta\emptyset$max) 2.333 mm reached after 180 minutes. | |
| Area under curve (AUC) 126 cm². | |

TABLE 2

| Tablets coated with layers (b) and (c) | |
| --- | --- |
| TIME (minutes) | ΔØ (mm) |
| 0 | 0 |
| 10 | .75 |
| 30 | 2.375 |
| 45 | 3 |
| 60 | 2.5 |
| 90 | 2.625 |
| 120 | 2.625 |
| 150 | 2.75 |
| 180 | 2.75 |
| 210 | 2.25 |
| 240 | 2.75 |
| 270 | 2.75 |
| 300 | 2.125 |
| 330 | 1.125 |
| 360 | 1.5 |
| 390 | 1.375 |
| 420 | .25 |
| Maximum pupil diameter variation 3.0 mm reached after 45 minutes. In addition, a ΔØ value exceeding 2.0 mm was maintained until 300 minutes. Area under curve (AUC) 188 cm². | |

It is apparent that applying the impermeable and bioadhesive layers results in more rapid onset of the pharmacological effect, which is maintained for a longer time than with the same tablet uncoated.

**Claims**

1. A tablet for ophthalmic use with controlled release of the active substance, characterised by:
   - a matrix (a) of defined geometrical form containing the active substance and excipients and/or additives able to give the mixture the characteristics necessary for its compression;
   - a polymer coating (b) applied to a portion of the surface of said matrix such as to make the coated matrix portion impermeable or slowly permeable;
   - a possible layer (c) of bioadhesive polymer material applied to said coating (b).

2. A tablet as claimed in claim 1, characterised in that said active substance is pilocarpine, a beta-blocking agent, a steroid or non-steroid antiinflammatory, an antibiotic or an antiviral, said substances being used singularly or in association.

3. A tablet as claimed in claim 1, characterised in that said excipients are biocompatible polymer materials such as polyacrylic acid of various molecular weights, polyvinylalcohol of different molecular weights, hydroxypropylcellulose and hydroxymethylcellulose.

4. A tablet as claimed in claim 1, characterised in that said additives are paraffins, magnesium stearate and glycerol mono-bi-tripalmito-stearate.

5. A tablet as claimed in claim 1, characterised in that biocompatible polymer materials such as polymethylmethacrylates, ethylcellulose and hydroxypropylcellulose are used for the coating (b).

6. A tablet as claimed in claim 1, characterised in that said bioadhesive polymer materials are natural and/or synthetic polycarboxylic acids, hyaluronic acid, polyacrylic acid, alginic acid,carboxypoly-methylene and polygalacturonic acid.

7. A tablet as claimed in claim 1, characterised by being of cylindrical, lenticular or oblong shape.

8. A method for preparing tablets for ophthalmic use with controlled release of the active substance, comprising:
- a matrix (a) of defined geometrical form containing the active substance and excipients and/or additives able to give the mixture the characteristics necessary for its compression;
- a polymer coating (b) applied to a portion of the surface of said matrix such as to make the coated matrix portion impermeable or slowly permeable;
- a possible layer (c) of bioadhesive polymer material applied to said coating (b);
characterised in that said matrix (a) is prepared by compression to between 300 and 5000 kg/cm$^2$, and that said coating (b) and said layer (c) are applied by compression of the polymer material or by atomization or deposition of a solution or suspension of said material.

9. A method as claimed in claim 8, characterised in that said solution of polymer material is prepared using alcohols or ketones as solvents, with a polymer material content of between 1 and 40% by weight.

10. A method as claimed in claim 8, characterised in that said suspension of polymer material. is prepared using water as suspending agent, with a polymer material content of between 1 and 40% by weight.

11. A method as claimed in claim 8, characterised in that said atomization is effected by spraying said solution or suspension using an air jet or an airless gun, at ambient temperature.

12. A method as claimed in claim 8, characterised in that said deposition is effected by gravity fall, metering a defined quantity of said solution or suspension, at ambient temperature.


Claims for the following Contracting States: GR,ES

1. A method for preparing tablets for ophthalmic use with controlled release of the active substance, comprising:
- a matrix (a) of defined geometrical form containing the active substance and excipients and/or additives able to give the mixture the characteristics necessary for its compression;
- a polymer coating (b) applied to a portion of the surface of said matrix such as to make the coated matrix portion impermeable or slowly permeable;
- a possible layer (c) of bioadhesive polymer material applied to said coating (b);
characterised in that said matrix (a) is prepared by compression to between 300 and 5000 kg/cm$^2$, and that said coating (b) and said layer (c) are applied by compression of the polymer material or by atomization or deposition of a solution or suspension of said material.

2. A method as claimed in claim 1, characterised in that said solution of polymer material is prepared using alcohols or ketones as solvents, with a polymer material content of between 1 and 40% by weight.

3. A method as claimed in claim 1, characterised in that said suspension of polymer material is prepared using water as suspending agent, with a polymer material content of between 1 and 40% by weight.

4. A method as claimed in claim 1, characterised in that said atomization is effected by spraying said solution or suspension using an air jet or an airless gun, at ambient temperature.

5. A method as claimed in claim 1, characterised in that said deposition is effected by gravity fall, metering a defined quantity of said solution or suspension, at ambient temperature.

8

FIG. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 226 884  (P. COLOMBO et al.)<br>* page 3, line 17 - page 5, line 5; claims 1-23 * | | A 61 K    9/06 |
| A | US-A-3 811 444  (JORGE HELLER et al.)<br>* Column 9, line 3 - column 10, line 10; column 13, line 34 - column 14, line 54; figure 5; claim 1-11 * | | |
| A | EP-A-0 033 042  (MERCK & CO. INC.)<br>* page 6, line 34 - page 10, line 36 * | | |
| A | EP-A-0 077 261  (MERCK & CO. INC.)<br>* page 2, line 18 - page 7, line 19 * | | |
| A | WO-A-8 502 092  (BIO-MIMETICS INC.)<br>* page 7, line 6 - page 8, line 7; page 11, line 24 - page 13, line 13; page 14, line 31 - page 28, line 21 * | | |
| A | FR-A-2 514 642  (SANDOZ S.A.)<br>* page 4, line 22 - page 10, line 12 * | | TECHNICAL FIELDS SEARCHED (Int. Cl.4)<br><br>A 61 K |

The present search report has been drawn up for all claims

| Place of search | Date of completion of the search | Examiner |
|---|---|---|
| THE HAGUE | 22-02-1989 | TZSCHOPPE,D.A. |

EPO FORM 1503 03.82 (P0401)